Europäisches Patentamt

European Patent Office

Office européen des brevets

(11) Numéro de publication : **0 397 828 B1**

(19)

(12) **FASCICULE DE BREVET EUROPEEN**

(45) Date de publication du fascicule du brevet :
24.05.95 Bulletin 95/21

(51) Int. Cl.[6] : **G01N 33/546,** G01N 33/58,
// G01N33/569

(21) Numéro de dépôt : **89912640.3**

(22) Date de dépôt : **03.11.89**

(86) Numéro de dépôt international :
**PCT/FR89/00573**

(87) Numéro de publication internationale :
**WO 90/05307 17.05.90 Gazette 90/11**

(54) **Méthode d'agglutination pour l'analyse de plusieurs ligands.**

(30) Priorité : **04.11.88 FR 8814420**

(43) Date de publication de la demande :
**22.11.90 Bulletin 90/47**

(45) Mention de la délivrance du brevet :
**24.05.95 Bulletin 95/21**

(84) Etats contractants désignés :
**AT BE CH DE FR GB IT LI LU NL SE**

(56) Documents cités :
**EP-A- 126 450
DE-A- 3 811 566
FR-A- 2 627 286
US-A- 4 499 052**

(56) Documents cités :
**PATENT ABSTRACTS OF JAPAN, vol. 10, no.
322 (P-511)(2378), 31 octobre 1986
BIOLOGICAL ABSTRACTS, vol. 82, no. 5, 1986,
Philadelphia, PA (US); P.K. BJORNSEN, p.
AB-574, no. 45417**

(73) Titulaire : **CHEMUNEX
41 rue du 11 novembre 1918
F-94700 Maisons-Alfort (FR)**

(72) Inventeur : **VAN HOEGAERDEN, Michel
29, route de Bombon
Bréau
F-77720 Mormant (FR)**

(74) Mandataire : **Orès, Bernard et al
Cabinet ORES
6, Avenue de Messine
F-75008 Paris (FR)**

EP 0 397 828 B1

## Description

La présente invention est relative à un procédé de détection et/ou de dosage sensible et spécifique dans un échantillon liquide, semi-liquide ou pâteux, de plusieurs ligands à analyser à l'aide d'une réaction d'agglutination appropriée.

Il est connu de détecter et de doser des substances solubles et notamment des peptides ou des protéines par une réaction d'agglutination ou une réaction d'inhibition d'agglutination.

La réaction d'agglutination est la conséquence directe de la fixation d'un anticorps agglutinant sur une cellule, par exemple et fait ainsi appel à des complexes antigènes-anticorps ou hormones-récepteurs présentant des propriétés d'agglutination.

Néanmoins, il faut rappeler que bien que ces réactions soient aisées à réaliser, leur interprétation à l'oeil nu est la plupart du temps délicate et nécessite la présence d'une quantité importante de substance à doser dans l'échantillon, pour que l'interprétation soit aisée.

Une réaction d'agglutination peut être envisagée dans différents contextes biologiques ; en particulier dans le domaine immunologique, l'agglutination est la visualisation de la formation d'un complexe.

Il existe des méthodes dites directes (complexe antigène/anticorps), des méthodes dites indirectes ou des méthodes d'inhibition par compétition ; on connait, également, des méthodes de dosage dites de type "sandwich" ou bi-site ; dans ces méthodes de type "sandwich", deux anticorps sont utilisés, l'un pour capturer l'antigène, l'autre pour révéler sa présence, si cet antigène est effectivement présent et a été capté par le premier anticorps. En général, le premier anticorps est fixé sur un support solide (plaque, bille...), le deuxième anticorps étant conjugué à un "révélateur" de réaction tel que fluorochrome, radio-isotope, enzyme.

Dans une réaction d'agglutination, des globules rouges recouverts d'un antigène servent à détecter les anticorps spécifiques correspondant (hémagglutination). Une extension des méthodes d'agglutination a consisté à utiliser des billes en plastique : en cas de réaction positive, on observe l'apparition d'une "granulation" macroscopique (agglutination passive). Une procédure inverse a également été décrite : les anticorps sont fixés sur les billes et servent à détecter les antigènes correspondants. La sensibilité d'une telle méthode est élevée, mais, comme on l'a déjà mentionné ci-dessus, l'interprétation du résultat n'est pas objective et est variable en fonction de l'observateur, l'importance de l'agglutination étant fonction de la quantité d'antigène présent pour une certaine gamme de concentrations de cet antigène.

Afin de pallier cet inconvénient des méthodes d'agglutination et afin de les rendre quantitatives, un certain nombre d'auteurs ont proposé le comptage des particules non agglutinées à l'aide d'un compteur de particules approprié notamment un compteur de cellules sanguines.

MASSON & al. (Particle Counting Immunoassay, Meth. Enzymol., 1981, 74, 106-139) proposent un procédé de dosage d'antigènes divers (protéines, peptides ...), d'anticorps, d'haptènes et d'immuncomplexes à l'aide d'une réaction d'agglutination entre des particules de latex de 0,8 μm de diamètre recouvertes d'anticorps ou d'antigènes appropriés et lesdits antigènes ou anticorps ou haptènes ou immuncomplexes , et le comptage des particules non agglutinées à l'aide d'un compteur de particules, notamment un compteur de globules rouges qui est un détecteur de lumière diffractée aux petits angles. Le comptage des particules non agglutinées permet d'évaluer l'étendue de la réaction d'agglutination et, en conséquence, la quantité de produit à doser.

Le détecteur est paramétré de manière à ignorer électroniquement les particules dont le diamètre est inférieur à 0,6 μm et les particules dont le diamètre est supérieur à 1,2 μm (particules agglutinées).

MASSON a décrit les différents éléments de cette méthode dans un certain nombre de brevets :
- le Brevet US 4 062 935 (1977), revendique une méthode de détection de complexes antigène/anticorps, qui comprend l'addition à l'échantillon d'une solution de facteur rhumatoïde (RF) et d'un matériel qui agglutine au contact du RF puis détection de l'importance de l'agglutination dudit matériel comparée à un mélange standard dudit matériel et de RF ; ledit matériel est constitué de particules inertes recouvertes d'immunoglobulines, lesdites particules étant, plus particulièrement, en polystyrène. Ce Brevet décrit également une méthode de détection de la présence ou de l'absence dans un échantillon de fluide biologique de complexes anticorps/antigène comprenant l'addition à l'échantillon d'une quantité connue de RF et d'une quantité connue de particules de latex recouvertes avec des IgG caractérisé en ce que le RF se lie à n'importe quel complexe antigène/anticorps présents, le reste du RF entraînant l'agglutination des particules de latex ; puis détermination de la quantité dudit reste de RF par comptage des particules de latex agglutinés et comparaison du résultat avec une courbe standard.
- le Brevet US 4 138 213 (1979) décrit une méthode automatisée de détermination d'une quantité d'un anticorps ou d'un antigène dans un échantillon de fluide biologique, comprenant plusieurs étapes et notamment une étape de comptage du nombre de particules non agglutinées.
- le Brevet US 4 162 895 (1979) décrit une méthode de détection d'antigènes, d'anticorps ou de complexes d'antigènes/anticorps, qui met en oeuvre un réactif à base de sérum de souris ; ce réactif se combine seulement avec les complexes antigènes/anticorps et est utilisé dans le cadre des réactions d'agglutination, par exemple une méthode de détection d'anticorps qui comprend l'addition à l'échantillon d'une quantité connue de particules recouvertes des antigènes correspondant auxdits anticorps puis comptage du nombre de particules non agglutinées et calcul de la quantité d'anticorps présents dans l'échantillon.

- le Brevet US 4 184 849 (1980) décrit une méthode de détection d'antigènes et d'anticorps dans un échantillon liquide par mise en oeuvre d'une réaction d'agglutination appropriée et détection de l'importance de l'agglutination, qui permet de déterminer la quantité d'anticorps ou d'antigènes présents dans l'échantillon ; on introduit dans le liquide deux réactifs sous forme de particules qui agglutinent ensemble mais dont l'agglutination est inhibée par l'anticorps ou l'antigène éventuellement présent.
- le Brevet US 4 397 960 (1983) décrit une méthode de dosage d'un antigène par une réaction d'agglutination. Le dosage consiste en la détermination de l'importance de l'agglutination des particules de latex dans la solution et ainsi permet la détermination de la quantité d'antigène dans l'échantillon ; l'appréciation de l'agglutination est effectuée par comptage sélectif des particules de latex agglutinées et non agglutinées.
- Le résumé paru dans Patent Abstracts of Japan, vol. 10, n° 322 (P-511) (2378), 31 octobre 1986, correspondant à la Demande JP-A-61 128 169, décrit une méthode d'analyse immunologique d'un ligand qui permet la mesure du degré d'agrégation de particules fluorescentes recouvertes d'un antigène ou d'un anticorps approprié, par rapport à l'augmentation de l'intensité de fluorescence.

Ces différentes méthodes présentent néanmoins un certain nombre d'inconvénients :

- il est nécessaire de traiter l'échantillon de manière à éliminer toute particule qui pourrait constituer un faux positif ; pour ce faire, il est nécessaire de faire subir à l'échantillon un certain nombre d'opérations physiques ou chimiques telles que l'utilisation de tampons particuliers, filtration, centrifugation ;
- la numération des particules agglutinées n'est possible que dans des milieux limpides ;
- par ailleurs, le comptage par diffraction aux petits angles ne permet pas d'avoir une bonne sensibilité si l'on essaie de compter les particules agglutinées (pics non séparés et donc difficilement interprétables) ;
- de plus, elles ne permettent pas la détection simultanée de plusieurs ligands.

La Demanderesse s'est en conséquence donné pour but de pourvoir à un procédé ne présentant pas les inconvénients des méthodes d'agglutination décrites dans l'Art antérieur.

On entend par antiligand, une structure moléculaire capable de reconnaître et de lier spécifiquement la/les substances organiques, biologiques ou médicamenteuses à rechercher, appelées ci-après ligands.

La présente invention a pour objet un procédé de détection et/ou de dosage de plusieurs ligands à analyser, dans un échantillon liquide, semi-liquide ou pâteux, par une méthode d'agglutination directe, indirecte ou d'inhibition d'agglutination par compétition, en mettant en oeuvre des structures fluorescentes, dénommées ci-après billes, aptes à fixer un antiligand approprié, lequel procédé est caractérisé en ce que, pour le dosage spécifique et simultané d'au moins deux ligands :

- l'on met en contact l'échantillon à analyser avec au moins deux populations différentes de billes fluorescentes, les billes constitutives de chaque population présentant des caractéristiques identiques en ce qui concerne leur longueur d'onde d'émission, leur diamètre, leur densité de fluorochromes et leur nombre d'antiligands fixés à leur surface, et
- l'on analyse ledit échantillon contenant lesdites billes à l'aide d'un moyen de mesure à un paramètre unique de mesure de fluorescence par ligand, pour détecter et/ou compter simultanément le nombre de billes non agglutinées, le nombre de billes agglutinées, le nombre d'agrégats de billes, et pour chaque agrégat, le nombre de billes qu'il comprend.

On entend par population homogène de billes fluorescentes, un ensemble de billes identiques, c'est-à-dire dont la longueur d'onde d'émission, le diamètre et/ou la densité de fluorochrome sont identiques, ainsi que le/les antiligands fixés à leur surface.

Selon un mode de mise en oeuvre avantageux du procédé conforme à l'invention, lesdites billes comprennent au moins deux antiligands fixés à leur surface.

Selon un autre mode de mise en oeuvre avantageux du procédé conforme à l'invention, lesdites billes ont un diamètre compris entre 0,05 μm et 10 μm.

Les billes fluorescentes conformes au système de détection peuvent être, notamment, des billes de latex, des billes en polyacrylamide, des billes de cellulose, des billes en polystyrène, des billes d'agarose, des billes en PVC, des billes de verre.

Selon encore un autre mode de mise en oeuvre avantageux du procédé conforme à l'invention, le/les antiligands sont choisis dans le groupe qui comprend des antigènes, des anticorps, des récepteurs, des lectines, des protéines de fixation aux immunoglobulines de type A ou G et des avidines, spécifiques de la/les substances ou ligands à rechercher.

Selon une disposition avantageuse de ce mode de mise en oeuvre, les anticorps sont des anticorps monoclonaux.

Selon une autre disposition avantageuse de ce mode de mise en oeuvre, les anticorps sont des anticorps polyclonaux.

Les quantités totales de lumières fluorescentes varient en fonction du nombre de billes agglutinées dans chaque agrégat ou agglutinat et permet ainsi, à l'aide du moyen de détection de fluorescence, la discrimination du nombre de billes fluorescentes présentes dans chaque agglutinat et de leur taille.

Une telle discrimination permet de connaître le nombre de doublets, triplets, quadruplets, etc..., formés et donne, de ce fait, à ce procédé de dosage, une sensibilité et une précision de mesure nettement accrues.

Les substances à analyser qui peuvent, notamment, être détectées par le procédé conforme à l'invention, comprennent au moins deux ligands susceptibles de fixer un antiligand, par exemple des substances organiques, biologiques ou médicamenteuses et notamment des antigènes, des anticorps, des haptènes et des immuncomplexes, des hormones, des récepteurs membranaires, des lectines, des protéines de fixation des immunoglobulines de type A ou G, ou des avidines.

Selon encore un autre mode de mise en oeuvre avantageux du procédé conforme à l'invention, le moyen de mesure de fluorescence est choisi dans le groupe qui comprend le cytomètre en flux, l'analyseur d'image et un système de balayage laser.

Le procédé de détection et de comptage conforme à l'invention, présente un certain nombre d'avantages tant économiques (manipulations peu importantes, temps de comptage très rapide) que techniques :

- il permet, en particulier, le comptage des billes agglutinées et non agglutinées dans un milieu turbide ou visqueux ;
- il permet le comptage des billes agglutinées et non agglutinées à l'unité près ;
- il est, de ce fait, très sensible, ce qui permet de doser de très petites quantités d'une substance recherchée ;
- les billes fluorescentes utilisées évitent les interférences avec d'autres particules éventuellement présentes et n'obligent ni à une séparation physique de particules éventuellement présentes dans l'échantillon à doser ni à des étapes de lavage, ce qui constitue un dosage homogène ; en effet, l'utilisation du système de détection conforme à l'invention permet de travailler dans un milieu complexe contenant une masse importante d'autres particules, le nombre d'éléments autofluorescents étant très faible et pouvant être ignorés par le moyen de détection, notamment par un choix judicieux des filtres optiques ;
- il permet de discriminer les billes non agglutinées, les doublets, les triplets, etc..., car il permet d'effectuer une mesure absolue de l'intensité et de la longueur d'onde de fluorescence de chaque particule ou agrégat de particules, un à un.

On peut notamment, et ce, de manière non limitative utiliser le cytomètre en flux décrit dans la demande de Brevet français n° 88 02937 (FR-A-2 628 531) : il permet de passer des échantillons de volumes importants, possède un système de lavage automatique entre chaque échantillon et est conçu pour une utilisation en routine ; il est, de plus, capable de détecter chaque bille agglutinée ou non et de les compter individuellement, jusqu'à une fréquence de 3 000 par seconde.

La forme de la cellule en flux est choisie pour réduire à leur minimum les forces hydrodynamiques d'arrachage.

La présente invention a, en outre, pour objet un kit ou coffret prêt à l'emploi pour la mise en oeuvre du procédé de détection et/ou de dosage conforme à l'invention, caractérisé en ce qu'il comprend au moins deux populations de billes sur lesquelles est fixé au moins un antiligand approprié, éventuellement associé à des tampons appropriés.

La présente invention a, également, pour objet un kit ou coffret prêt à l'emploi pour la mise en oeuvre du procédé de détection et/ou de dosage conforme à l'invention, caractérisé en ce qu'il comprend au moins deux populations de billes, sur lesquelles sont fixés au moins deux antiligands différents appropriés.

Le procédé conforme à l'invention, appliqué à l'analyse d'un carbohydrate complexe, est caractérisé en ce que l'on met en contact l'échantillon à analyser avec au moins deux populations de billes fluorescentes sur lesquelles sont fixées des lectines de spécificité différentes.

Egalement conformément à l'invention, ledit procédé, appliqué à l'analyse d'un matériel nucléique pour rechercher la présence de séquences spécifiques, est caractérisé en ce que l'on met en contact l'échantillon à analyser avec au moins deux populations de billes fluorescentes sur lesquelles sont fixées des sondes nucléiques s'hybridant avec des séquences complémentaires sur un même gène.

Outre les dispositions qui précèdent, l'invention comprend encore d'autres dispositions qui ressortiront de la description qui va suivre, qui se réfère à des exemples de mise en oeuvre du procédé conforme à l'invention.

Il doit être bien entendu, toutefois, que ces exemples sont donnés uniquement à titre d'illustration de l'objet de l'invention, dont ils ne constituent en aucune manière une limitation.

Le procédé conforme à l'invention comprend la formation de complexes par contact de l'échantillon avec des billes fluorescentes sur lesquelles est fixé au moins un antiligand approprié, dans des conditions convenables à l'apparition d'une agglutination ou à l'inhibition d'une agglutination, puis la détection des billes agglutinées et non agglutinées.

Dans une mise en oeuvre préférée du procédé, des populations de billes fluorescentes portant des antiligands complémentaires du ligand à doser sont mélangées en nombre égal, environ 50 000 par ml. La solution ou suspension à tester est ajoutée sous un volume pouvant varier de 3 à 1000 µl ou plus. Une incubation à 37°C accélère la formation de la liaison ligand-antiligand ; la durée de cette incubation dépend essentiellement de la constante d'affinité des antiligands mis en oeuvre ; la sensibilité finale du test en dépendra également.

Le procédé conforme à l'invention, permet la détection d'une seule bille fluorescente. Si l'on en compte deux, on peut simultanément déterminer si elles sont séparées ou agglutinées, et dans le cas ou elles sont agglutinées, si elles ont les mêmes caractéristiques ou non. La sensibilité de ce procédé permet, ainsi, théoriquement, de mesurer la présence d'une seule molécule de ligand, si celle-ci parvient à agglutiner deux billes fluorescentes, de mêmes caractéristiques spectrales ou non.

**Exemple 1** : Préparation et mode d'utilisation de billes fluorescentes :

1) <u>Préparation des billes fluorescentes recouvertes d'un antiligand</u> (système de détection)

Dans un premier temps, le système de détection est préparé par couplage des particules fluorescentes avec le réactif approprié selon des méthodes connues.

- Couplage covalent de particules fluorescentes carboxylées avec des anticorps polyclonaux de lapin anti-entérotoxine A :

    a - Préparation des billes (Fluoresbrite® - Polysciences) :

        . 0,5 g de billes en suspension à 2,5 % sous 4 ml de tampon borate ($10^{-2}$ M d'acide borique + 0,15 M NaCl, pH 8,1) ;

        . préparer une solution contenant 80 µmoles d'hexamethylène-diamine dans 1 ml de tampon borate ;

        . y ajouter les 4 ml de billes ;

        . ajouter la carbodiimide à 0,05 M ;

        . incuber une nuit à 4°C ;

        . laver avec le tampon borate.

    b - Préparation de l'anticorps :

        . dialyser les anticorps contre du tampon phosphate 0,1 M, pH 6 ;

        . prévoir 2/3 d'anticorps pour 1/3 de billes ;

        . ajouter 200 µm de $NaIO_4$ 0,15 M ;

        . incuber 3 heures à 4°C à l'obscurité ;

        . ajouter du glycérol à une concentration finale de 0,015 M ;

        . incuber 30 minutes à température ambiante ;

        . dialyser contre le tampon phosphate pH 6 ;

    c - Couplage proprement dit:

        . ajouter les billes et les anticorps en présence de 5 ml de tampon borate ;

        . incuber 16 heures à 4°C sous agitation ;

        . ajuster le pH à 8,8 ;

        . stabiliser avec $NaBH_4$ (5 mg dans l'essai) ;

        . incuber 5 heures à 4°C ;

        . laver avec du PBS-BSA à 0,5 % - Tween 0,1 %.

- Couplage non covalent de particules fluorescentes carboxylées avec des anticorps polyclonaux de lapin anti-entérotoxine A :

    a - Dans un tube en verre :

        . 100 µl d'une solution d'anticorps à 2,5 mg/ml ;

        . 100 µl de tampon Glycine (Meth. Enzymol. <u>74</u>, 111) ;

        . 5 µl de billes diluées au 1/10 ;

    b - Agitation pendant 30 minutes à température ambiante ;

    c - Rinçage : 3 fois dans un tampon PBS-BSA à 0,5 % - Tween à 0,1 % ;

d - Reprendre dans 100 µl de PBS.

2) <u>Détection de la substance recherchée :</u>

a) on utilise un système de détection tel que préparé en 1) ;

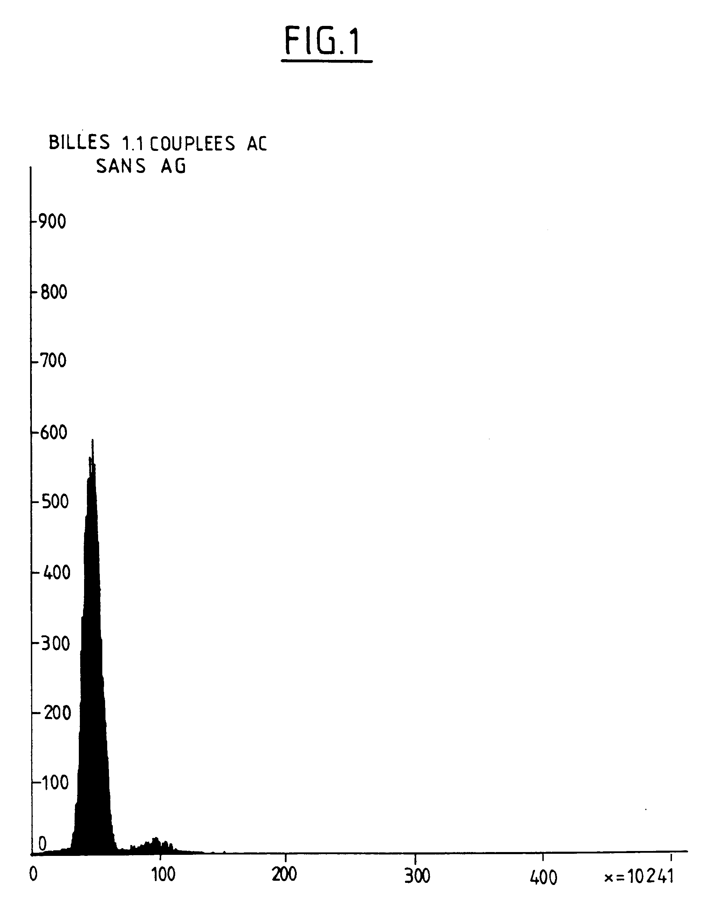

b) on mélange le système de détection avec une quantité d'antigène ; on incube 2 heures à 37° et on détecte les billes agglutinées et non agglutinées ; les résultats sont présentés sur le tableau I et sur la figure 1.

Le tableau I, ci-après, montre un essai comparatif entre un témoin négatif (billes recouvertes d'anticorps mais absence d'entérotoxine) pour lequel on obtient 10,5 % de billes agglutinées (figure 1 et colonne A du tableau I) et

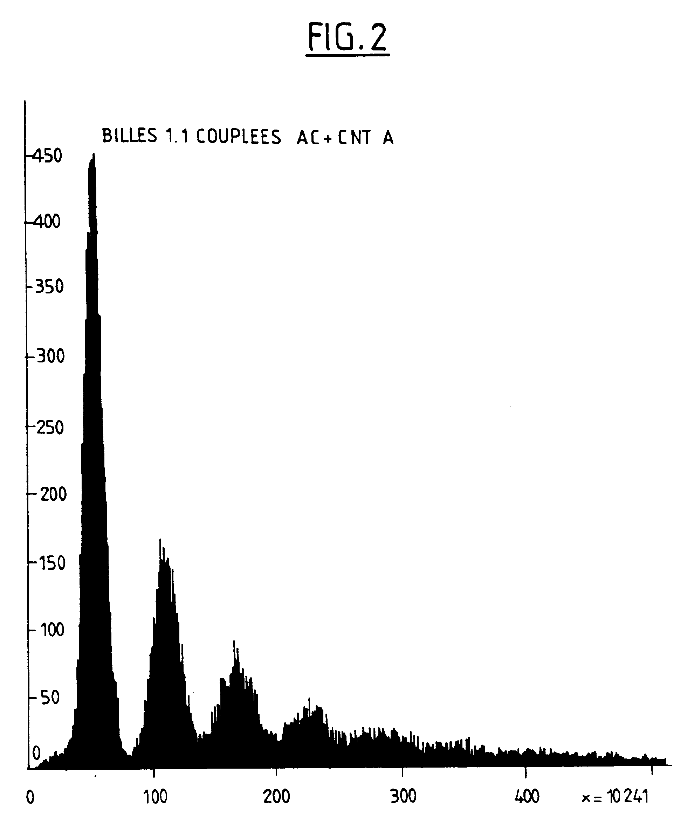

- une détection d'entérotoxine A, par le procédé conforme à l'invention, (figure 2 et colonne B du tableau I), pour lequel on obtient 85 % de billes agglutinées, en présence d'un échantillon contenant 10 ng d'entérotoxine A ; l'agglutination est proportionnelle à la concentration en entérotoxine A dans une gamme de concentrations donnée.

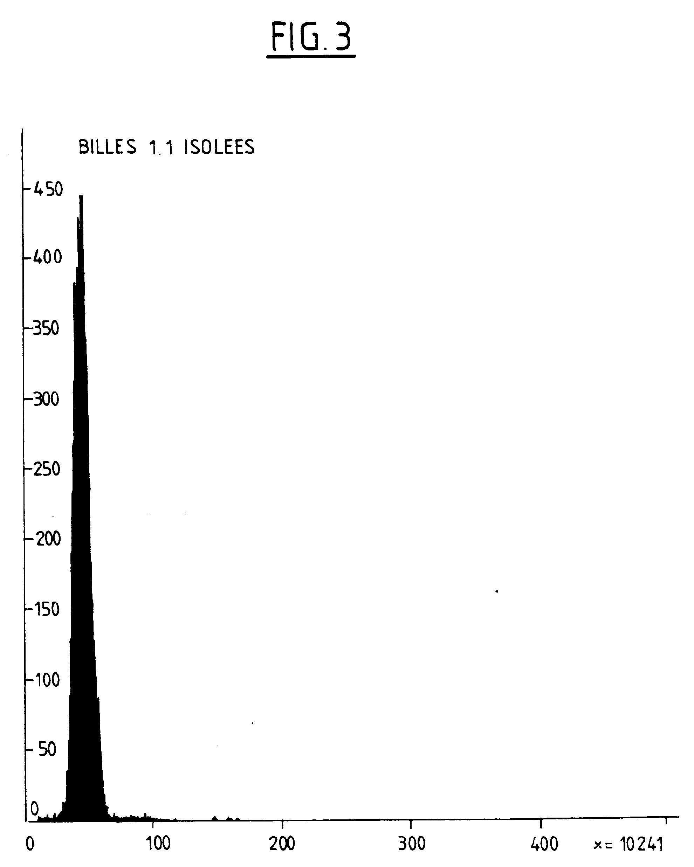

Dans les figures 1, 2 et 3, on a en abscisse, la mesure relative de fluorescence (canal) le numéro du canal et en ordonnée, le nombre de particules ou d'agrégats de particules par canal.

La figure 1 montre le résultat obtenu avec des billes de latex couplées à des anticorps (témoin négatif) et correspond aux résultats mentionnés dans le tableau I, colonne A.

La figure 2 montre, sous forme d'histogramme, les résultats mentionnés dans le tableau I, colonne B.

La figure 3 montre le résultat obtenu avec des billes de latex fluorescentes non couplées à des anticorps (autre témoin négatif), mais en présence d'antigène. On observe une agglutination inférieure à 2 %.

## TABLEAU I

BILLES 1,1 µm CARBOXYLEES COUPLEES AVEC UN ANTICORPS POLYCLONAL DE LAPIN ANTI-ENTEROTOXINE A

| NOMBRE DE BILLES PAR AMAS | CANAL | BILLES 1,1µm + ANTICORPS A | | | BILLES 1,1µm + ANTICORPS + 10ng ENTEROTOXINE A (B) | | |
|---|---|---|---|---|---|---|---|
| | | EVENEMENT / HISTO | BILLES / HISTO | % | EVENEMENT / HISTO | BILLES / HISTO | % |
| 1 | 52.25 | 9475 | 9475 | 89,522 | 8312 | 8312 | 15,497 |
| 2 | 112.5 | 466 | 932 | 8,8057 | 4198 | 8396 | 15,654 |
| 3 | 168.75 | 59 | 177 | 1,6723 | 2714 | 8142 | 15,18 |
| 4 | 225 | | | | 1581 | 6324 | 11,791 |
| 5 | 281.25 | | | | 1145 | 5725 | 10,674 |
| 6 | 337.5 | | | | 660 | 3960 | 7,3832 |
| 7 | 393.75 | | | | 495 | 3465 | 6,4603 |
| 8 | 450 | | | | 245 | 1960 | 3,6543 |
| 9 | 506.25 | | | | 180 | 1620 | 3,0204 |
| 10 | 562.5 | | | | 126 | 1260 | 2,3492 |
| 11 | 618.75 | | | | 122 | 1342 | 2,5021 |
| 12 | 675 | | | | 69 | 828 | 1,5438 |
| 13 | 731.25 | | | | 58 | 754 | 1,4058 |
| 14 | 787.5 | | | | 33 | 462 | 0,8614 |
| 15 | 843.75 | | | | 31 | 465 | 0,867 |
| 16 | 900 | | | | 11 | 176 | 0,3281 |
| 17 | 956.25 | | | | 12 | 204 | 0,3803 |
| 18 | 1012.5 | | | | 13 | 240 | 0,4475 |
| 19 | 1068.8 | | | | | | |
| SOMME | | 10000 | 10584 | 100 | 20005 | 53635 | 100 |

Le Tableau II, ci-après, montre les résultats obtenus avec un témoin négatif (billes recouvertes d'anticorps, mais en l'absence d'antigène correspondant) dans des conditions opératoires légèrement différentes (agita-

7

tion douce pendant 30 minutes seulement). On obtient, en ce cas, 1,6 % de billes agglutinées (bruit de fond et interférences très faibles).

TABLEAU II

| NOMBRE DE BILLES PAR AMAS | CANAL | BILLES 1,1 μm + ANTICORPS 30 MIN | | |
|---|---|---|---|---|
| | | EVENEMENT / HISTO | BILLES / HISTO | % |
| 1 | 56 | 10787 | 10787 | 98.395 |
| 2 | 112 | 88 | 176 | 1.6054 |
| 3 | 168 | | | |
| 4 | 224 | | | |
| 5 | 280 | | | |
| 6 | 336 | | | |
| 7 | 392 | | | |
| 8 | 448 | | | |
| 9 | 504 | | | |
| 10 | 560 | | | |
| 11 | 616 | | | |
| 12 | 672 | | | |
| 13 | 728 | | | |
| 14 | 784 | | | |
| 15 | 840 | | | |
| 16 | 896 | | | |
| 17 | 952 | | | |
| 18 | 1008 | | | |
| 19 | | | | |
| SOMME | | 10875 | 10963 | 100 |

**Exemple 2** : Etude de la stabilité des billes recouvertes d'anticorps appropriés.

Des billes recouvertes d'anticorps polyclonaux de lapin anti-entérotoxine A, telles que décrites dans l'exemple 1, sont utilisées une semaine après leur préparation ; on obtient les résultats tels que représentés dans le tableau III :
- en colonne A sont représentés les résultats concernant un témoin négatif billes recouvertes d'anticorps ;
- en colonne B sont représentés les résultats concernant un test de détection d'entérotoxine A, la quantité de celle-ci étant de 10 ng ; la lecture du résultat est réalisée après 20 minutes sous agitation douce ;
- en colonne C sont représentés les résultats concernant un test de détection d'entérotoxine A, la quantité de celle-ci étant de 50 ng ; la lecture du résultat est réalisée après 20 minutes sous agitation douce ;
- on observe une inhibition de l'agglutination des billes en cas d'excès d'antigène (50 ng contre 10 ng) : 51,3 % des billes non agglutinées contre 42,7 % des billes non agglutinées ; l'adjonction d'anticorps spécifiques libres rétablit l'agglutination des billes recouvertes d'anticorps qui sont saturés en antigène.

## TABLEAU III

### BILLES 1,1 µm CARBOXYLEES COUPLEES AVEC UN ANTICORPS POLYCLONAL DE LAPIN ANTI-ENTEROTOXINE A

| Nombre de billes par amas | Canal | A — BILLES 1,1 µm + ANTICORPS | | | B — BILLES 1,1 µm + ANTICORPS + 10ng ENTEROTOXINE A 20 MIN | | | C — BILLES 1,1 µm + ANTICORPS + 50ng ENTEROTOXINE A 20 MIN | | |
|---|---|---|---|---|---|---|---|---|---|---|
| | | Evenement/Histo | Billes/Histo | % | Evenement/Histo | Billes/Histo | % | Evenement/Histo | Billes/Histo | % |
| 1 | 56 | 7831 | 7831 | 92,119 | 11308 | 11308 | 42,7 | 11878 | 11878 | 51,29 |
| 2 | 112 | 158 | 316 | 3,717 | 2280 | 4560 | 17,22 | 1714 | 3428 | 14,80 |
| 3 | 168 | 118 | 354 | 4,164 | 777 | 2331 | 8,80 | 663 | 1989 | 8,58 |
| 4 | 224 | | | | 436 | 1744 | 6,58 | 313 | 1252 | 5,40 |
| 5 | 280 | | | | 239 | 1195 | 4,51 | 196 | 980 | 4,23 |
| 6 | 336 | | | | 163 | 978 | 3,69 | 113 | 678 | 2,92 |
| 7 | 392 | | | | 127 | 889 | 3,35 | 68 | 476 | 2,05 |
| 8 | 448 | | | | 80 | 640 | 2,41 | 52 | 416 | 1,79 |
| 9 | 504 | | | | 72 | 648 | 2,44 | 44 | 396 | 1,71 |
| 10 | 560 | | | | 41 | 410 | 1,54 | 30 | 300 | 1,29 |
| 11 | 616 | | | | 41 | 451 | 1,70 | 26 | 286 | 1,23 |
| 12 | 672 | | | | 26 | 312 | 1,17 | 22 | 264 | 1,14 |
| 13 | 728 | | | | 16 | 208 | 0,78 | 12 | 156 | 0,67 |
| 14 | 784 | | | | 18 | 252 | 0,95 | 10 | 140 | 0,60 |
| 15 | 840 | | | | 9 | 135 | 0,51 | 10 | 150 | 0,64 |
| 16 | 896 | | | | 8 | 128 | 0,48 | 11 | 176 | 0,76 |
| 17 | 952 | | | | 14 | 238 | 0,89 | 8 | 136 | 0,58 |
| 18 | 1008 | | | | 3 | 54 | 0,20 | 3 | 54 | 0,23 |
| SOMME | | 8107 | 8501 | 100 | 15658 | 26481 | 100 | 15173 | 23155 | 100 |

Dans la suite des exemples, on entend par billes fluorescentes vertes, des billes marquées à la coumarine et par billes fluorescentes rouges, des billes marquées à la phycoérythrine R.

**Exemple 3** : Analyse de la protection immune de la femme enceinte contre la toxoplasmose.

On prépare des billes fluorescentes recouvertes d'antigène toxoplasmique ainsi que des billes fluorescentes recouvertes d'anticorps anti-IgG et de billes recouvertes d'anticorps anti-IgM comme dans l'exemple 1 (1a).

L'objectif consiste à rechercher simultanément la présence d'anticorps anti-toxoplasme d'isotypes IgG et IgM. Des billes fluorescentes vertes sont recouvertes d'antigène toxoplasmique et sont ensuite incubées avec l'échantillon sanguin en présence de billes vertes recouvertes d'anticorps anti-IgG et de billes rouges recouvertes d'anticorps anti-IgM. La proportion de doublets verts/verts d'une part et verts/rouges d'autre part permettent de rechercher et de quantifier simultanément les anticorps IgG et IgM. Les agrégats à 3 billes ou plus sont analysés pour leurs composantes rouges et vertes. Par ailleurs, on établit une courbe de référence.

**Exemple 4** : Dosage rapide d'isomères ou d'épitopes dénaturés.

On prépare les billes fluorescentes recouvertes d'un antiligand approprié comme dans l'exemple 1.

Un anticorps monoclonal commun à tous les isomères ou reconnaissant toutes les formes natives et dénaturées d'une protéine recouvre des billes vertes. Un anticorps monoclonal spécifique de chaque forme modifiée est couplé à des billes soit vertes soit rouges. La recherche des agrégats vert/vert et vert/rouge donne simultanément la quantité totale d'antigène et la proportion des deux (ou plus, si l'on choisit plusieurs couleurs de billes) formes de la molécule.

**Exemple 5** : Recherche, analyse et dosage de carbohydrates complexes.

La fixation de lectines de spécificités différentes (par exemple concanavaline A pour $\alpha$-D-glucosyl et agglutinine de germe de blé pour $(\beta$-N-acétylglucosaminyl$)_n$ ou les acides sialiques) sur des billes de différentes couleurs, selon le protocole de l'exemple 1, permet de déterminer si les sucres reconnus par ces lectines sont portés par la même molécule (agglutination) ou par des molécules différentes (pas d'agglutination) ceci constitue également un outil de détermination de structures : on connaît en effet plusieurs dizaines de lectines différentes à ce jour.

**Exemple 6** : Recherche de séquences spécifiques d'acides nucléiques.

- Deux (ou plus) sondes nucléiques hybridant avec des séquences complémentaires sur un même gène sont couplées à des billes de couleurs différentes, selon le protocole de l'exemple 1.
- Le matériel nucléique, libéré des cellules, est mis en présence des diverses populations de billes fluorescentes dans des conditions d'hybridation.
- La recherche des agrégats se fait par cytométrie en flux, par analyse d'images ou par balayage laser. La présence d'agrégats constitués de billes de même couleur indique la présence de séquences répétitives sur le même gène ou le même chromosome alors que les agrégats constitués de billes de couleurs différentes indique l'existence complémentaire des deux (ou plus) séquences spécifiques recherchées.

## Revendications

1. Procédé de détection et/ou de dosage de plusieurs ligands à analyser, dans un échantillon liquide, semiliquide ou pâteux, par une méthode d'agglutination directe, indirecte ou d'inhibition d'agglutination par compétition, en mettant en oeuvre des structures fluorescentes, dénommées ci-après billes, aptes à fixer un antiligand approprié, lequel procédé est caractérisé en ce que, pour le dosage spécifique et simultané d'au moins deux ligands :
   - l'on met en contact l'échantillon à analyser avec au moins deux populations différentes de billes fluorescentes, les billes constitutives de chaque population présentant des caractéristiques identiques en ce qui concerne leur longueur d'onde d'émission, leur diamètre, leur densité de fluorochromes et leur nombre d'antiligands fixés à leur surface, et
   - l'on analyse ledit échantillon contenant lesdites billes à l'aide d'un moyen de mesure à un paramètre unique de mesure de fluorescence par ligand, pour détecter et/ou compter simultanément le nombre de billes non agglutinées, le nombre de billes agglutinées, le nombre d'agrégats de billes, et pour chaque agrégat, le nombre de billes qu'il comprend.

2. Procédé selon la revendication 1, caractérisé en ce que lesdites billes comprennent au moins deux antiligands fixés à leur surface.

3. Procédé selon la revendication 1 ou la revendication 2, caractérisé en ce que lesdites billes ont un diamètre compris entre 0,05 μm et 10 μm.

4. Procédé selon l'une quelconque des revendications 1 à 3, caractérisé en ce que l'antiligand est choisi dans le groupe qui comprend des antigènes, des anticorps, des récepteurs, des lectines, des protéines de fixation aux immunoglobulines de type A ou G, des avidines, spécifiques des ligands à rechercher.

5. Procédé selon la revendication 4, caractérisé en ce que les anticorps sont des anticorps monoclonaux.

6. Procédé selon la revendication 4, caractérisé en ce que les anticorps sont des anticorps polyclonaux.

7. Procédé selon l'une quelconque des revendications 1 à 6, caractérisé en ce que le moyen de mesure de fluorescence est choisi dans le groupe qui comprend le cytomètre en flux, l'analyseur d'image et un système de balayage laser.

8. Kit ou coffret prêt à l'emploi pour la mise en oeuvre du procédé de détection et/ou de dosage selon l'une quelconque des revendications 1 à 7, caractérisé en ce qu'il comprend au moins deux populations de billes sur lesquelles est fixé au moins un antiligand approprié, éventuellement associé à des tampons appropriés.

9. Kit ou coffret prêt à l'emploi pour la mise en oeuvre du procédé de détection et/ou de dosage selon l'une quelconque des revendications 1 à 7, caractérisé en ce qu'il comprend au moins deux populations de billes, sur lesquelles sont fixés au moins deux antiligands différents appropriés.

10. Application du procédé selon la revendication 1 à l'analyse d'un carbohydrate complexe, caractérisée en ce que l'on met en contact l'échantillon à analyser avec au moins deux populations de billes fluorescentes sur lesquelles sont fixées des lectines de spécificités différentes.

11. Application du procédé selon la revendication 1 à l'analyse d'un matériel nucléique pour rechercher la présence de séquences spécifiques, caractérisée en ce que l'on met en contact l'échantillon à analyser avec au moins deux populations de billes fluorescentes sur lesquelles sont fixées des sondes nucléiques s'hybridant avec des séquences complémentaires sur un même gène.

**Patentansprüche**

1. Verfahren zum Nachweis und/oder zur Bestimmung von mehreren zu analysierenden Liganden in einer flüssigen, halbflüssigen oder pastösen Probe, durch ein direktes oder indirektes Agglutinationsverfahren oder ein kompetitives Agglutinationshemmverfahren, durch Einsetzen von fluoreszierenden Strukturen, nachstehend als Kügelchen bezeichnet, mit der Eignung, einen geeigneten Antiliganden zu fixieren, dadurch **gekennzeichnet**, daß zur spezifischen und gleichzeitigen Bestimmung von mindestens zwei Liganden
   - man die zu analysierende Probe mit mindestens zwei verschiedenen Populationen von fluoreszierenden Kügelchen in Kontakt bringt, wobei die Kügelchen, die jede Population bilden, identische Eigenschaften was ihre Emissionswellenlänge, ihren Durchmesser, ihre Fluorochromdichte und ihre Anzahl der auf ihrer Oberfläche fixierten Antiliganden betrifft, besitzen und
   - man die Probe, die die Kügelchen enthält, mit Hilfe einer Meßvorrichtung für einen einzigen Fluoreszenzmeßparameter pro Ligand analysiert, um gleichzeitig die Anzahl der nicht agglutinierten Kügelchen, die Anzahl der agglutinierten Kügelchen, die Anzahl der Aggregate von Kügelchen, und für jedes Aggregat, die Anzahl der Kügelchen, die es umfaßt, nachzuweisen und/oder zu zählen.

2. Verfahren nach Anspruch 1, dadurch **gekennzeichnet**, daß die Kügelchen mindestens zwei auf ihrer Oberfläche fixierte Antiliganden umfassen.

3. Verfahren nach Anspruch 1 oder 2, dadurch **gekennzeichnet**, daß die Kügelchen einen Durchmesser zwischen 0,05 μm und 10 μm besitzen.

4. Verfahren nach einem der Ansprüche 1 bis 3, dadurch **gekennzeichnet**, daß der Antiligand aus der Gruppe, umfassend Antigene, Antikörper, Rezeptoren, Lektine, Proteine zur Fixierung an Immunglobuline vom Typ A oder G, Avidine, welche für den zu bestimmenden Liganden spezifisch sind, ausgewählt wird.

5. Verfahren nach Anspruch 4, dadurch **gekennzeichnet**, daß die Antikörper monoclonale Antikörper sind.

6. Verfahren nach Anspruch 4, dadurch **gekennzeichnet**, daß die Antikörper polyclonale Antikörper sind.

7. Verfahren nach einem der Ansprüche 1 bis 6, dadurch **gekennzeichnet**, daß das Mittel zur Messung der Fluoreszenz aus der Gruppe, umfassend ein Durchflußzytometer, einen Bildanalysator und ein System zur Laserabtastung, ausgewählt wird.

8. Gebrauchsfertiges Kit oder Reagenszusammenstellung zur Durchführung des Nachweis- und/oder Bestimmungsverfahrens nach einem der Ansprüche 1 bis 7, dadurch **gekennzeichnet**, daß es mindestens zwei Populationen von Kügelchen, auf denen mindestens ein geeigneter Antiligand fixiert ist, gegebenenfalls zusammen mit geeigneten Puffern, umfaßt.

9. Gebrauchsfertiges Kit oder Reagenszusammenstellung zur Durchführung des Verfahrens zum Nachweis und/oder zur Bestimmung nach einem der Ansprüche 1 bis 7, dadurch **gekennzeichnet**, daß es mindestens zwei Populationen an Kügelchen umfaßt, auf denen mindestens zwei verschiedene geeignete Antiliganden fixiert sind.

10. Verwendung des Verfahrens nach Anspruch 1 zur Analyse eines komplexen Kohlenhydrats, dadurch **gekennzeichnet**, daß man die zu analysierende Probe mit mindestens zwei Populationen an fluoreszierenden Kügelchen in Kontakt bringt, auf denen Lektine mit verschiedenen Spezifitäten fixiert sind.

11. Verwendung des Verfahrens nach Anspruch 1 zur Analyse eines Nucleinsäurematerials zum Absuchen auf das Vorhandensein spezifischer Sequenzen, dadurch **gekennzeichnet**, daß man die zu analysierende Probe mit mindestens zwei Populationen an fluoreszierenden Kügelchen in Kontakt bringt, auf denen Nucleinsäuresonden fixiert sind, die mit den komplementären Sequenzen auf einem gleichen Gen hybridisieren.

## Claims

1. Method for detecting and/or assaying several ligands to be analysed, in a liquid, semi-liquid or pasty sample, by a process of direct or indirect agglutination or by a process of competitive inhibition of agglutination, using fluorescent structures, hereinafter referred to as beads, which are capable of binding a suitable antiligand, which method is characterized in that, for the specific and simultaneous assay of at least two ligands:
   - the sample to be analysed is placed in contact with at least two different populations of fluorescent beads, the beads constituting each population having identical characteristics as regards their transmission wavelength, their diameter, their fluorochrome density and the number of antiligands bound to their surface, and
   - the said sample containing the said beads is analysed using a measuring means having a single parameter for measuring ligand fluorescence, in order simultaneously to detect and/or to count the number of non-aggregated beads, the number of aggregated beads, the number of bead aggregates and, for each aggregate, the number of beads it contains.

2. Method according to Claim 1, characterized in that the said beads contain at least two antiligands bound to their surface.

3. Method according to Claim 1 or Claim 2, characterized in that the said beads have a diameter between 0,05 $\mu$m and 10 $\mu$m.

4. Method according to any one of Claims 1 to 3, characterized in that the antiligand is chosen from the group which comprises antigens, antibodies, receptors, lectins, proteins which bind to immunoglobulin A or G, and avidins, which are specific for the ligands to be detected.

5. Method according to Claim 4, characterized in that the antibodies are monoclonal antibodies.

6. Method according to Claim 4, characterized in that the antibodies are polyclonal antibodies.

7. Method according to any one of Claims 1 to 6, characterized in that the means for measuring the fluorescence is chosen from the group which comprises the flow cytometer, the image analyser and a laser scanning system.

8. Ready-to-use kit or requisite for the implementation of the detection and/or assay method according to any one of Claims 1 to 7, characterized in that it contains at least two populations of beads on which at least one suitable antiligand is bound, optionally combined with suitable buffers.

9. Ready-to-use kit or requisite for the implementation of the detection and/or assay method according to any one of Claims 1 to 7, characterized in that it contains at least two populations of beads, on which at least two different, suitable ligands are bound.

10. Application of the method according to Claim 1 to the analysis of a complex carbohydrate, characterized in that the sample to be analysed is placed in contact with at least two populations of fluorescent beads on which lectins of different specificities are bound.

11. Application of the method according to Claim 1 to the analysis of a nucleic acid material in order to detect the presence of specific sequences, characterized in that the sample to be analysed is placed in contact with at least two populations of fluorescent beads on which nucleic acid probes which hybridize with complementary sequences within the same gene are bound.

# FIG.1

BILLES 1.1 COUPLEES AC
SANS AG

# FIG.2

BILLES 1.1 COUPLEES  AC + CNT  A

# FIG. 3

BILLES 1.1 ISOLEES

x = 10241